# EUROPÄISCHE PATENTSCHRIFT

(11) **EP 2 016 977 B1**
(45) Veröffentlichungstag und Bekanntmachung des Hinweises auf die Patenterteilung: **20.06.2012**
(21) Anmeldenummer: 08012117.1
(22) Anmeldetag: 04.07.2008
(51) Int. Cl.: A61N 5/06

(54) **Lichttherapievorrichtung**
Light therapy device
Dispositif de luminothérapie

(30) Priorität: 17.07.2007 DE 102007033292
(43) Veröffentlichungstag der Anmeldung: 21.01.2009
(73) Patentinhaber: Bartenbach Holding GmbH, 6071 Aldrans (AT)
(72) Erfinder: Bartenbach, Christian, 6071 Aldran / Innsbruck (AT)
(74) Vertreter: Thoma, Michael

(56) Entgegenhaltungen:
- DE-A1- 4 433 291
- DE-A1- 19 606 396
- DE-C- 169 607
- DE-U1- 8 717 148
- FR-A- 2 639 239
- US-A- 6 053 936
- US-A1- 2006 217 789

## Beschreibung

Die vorliegende Erfindung betrifft eine Lichttherapievorrichtung zur Beeinflussung des Serotoninspiegels, mit einer Lichtabgabevorrichtung zur Abgabe von über das menschliche/tierische Auge aufnehmbaren Lichts, wobei die Lichtabgabevorrichtung eine Lichtkabine mit einer als Leuchte ausgebildeten Kabinenwand umfasst die das abgegebene Licht auf ein Kabinenzentrum richtet in dem ein Therapieplatz vorgesehen ist, wobei die Kabinenwand vom Therapieplatz aus betrachtet ein Sichtfeld über einen Raumwinkel von zumindest ¾ π umschließt.

Der Einfluss von Licht auf den menschlichen Organismus und seinen biologischen Rhythmus gilt inzwischen als unbestritten. Die Lichtwirkung hat unmittelbar Einfluss auf die Serotoninproduktion. Serotoninmangel wird mit Auftreten von Depressions-erscheinungen in Zusammenhang gebracht.

Um eine spürbare Wirkung auf die Serotoninproduktion zu haben, muss das auf die Pupille des Auges auffallende Licht eine gewisse Intensität haben bzw. wird als Wirkgröße der Faktor Lichtstromfluss, d.h. der auf die Netzhaut auffallende Lichtstrom multipliziert mit der Wirkungszeit definiert. Für verschiedene Lichtarten gibt es dabei erfahrungsgemäß verschiedene, je nach Spektrum unterschiedliche Wirkungsfaktoren. Derzeit übliche Empfehlungen nennen als Therapiewerte 10000 Ix bei einer Wirkungszeit von einer halben Stunde, 5000 Ix bei einer Wirkungszeit von einer Stunde und 2500 Ix bei einer Wirkungszeit von zwei Stunden.

Bekannt sind hierbei Lichttherapievorrichtungen in Form mobiler, bildschirmartiger Geräte, die vor der zu therapierenden Person aufgestellt werden. Ein solches Gerät ist beispielsweise unter dem Handelsnamen "Davita Lichtdusche Physiolight LD 220" bekannt, das ein etwa fernsehbildschirmgroßes Beleuchtungspaneel umfasst, das eine Beleuchtungsstärke von weit über 10000 Ix bereitstellt, so dass im vorgesehenen Abstand der zu therapierenden Person von dem Gerät von etwa einem halben Meter eine Beleuchtungsstärke von 10000 Ix auf der Pupille erzeugt wird.

Eine ähnliche Lichttherapievorrichtung in Form eines Fernseh-/Computermonitors zeigt die DE 20 2005 010 124 U1. Nachteilig bei derartigen vorbekannten Geräten sind die oftmals auftretenden Nebenwirkungen, insbesondere in Form von Kopfschmerzen. Zudem wird die Behandlung oftmals als unangenehm empfunden, da das Beleuchtungspaneel sozusagen dauerhaft anzustarren ist, um das Licht über die Pupille aufnehmen und eine genügende Wirkung erreichen zu können. Hiervon abgesehen sind die genannten Geräte oft ineffektiv durch eine Diffusstreuung des produzierten Lichts an einem das Beleuchtungspaneel abschließenden Diffusglases, wodurch ein wesentlicher Lichtstromanteil nicht ins Auge gestrahlt wird und hohe Verluste auftreten, die zu einer großen Wärmelast und hohem Stromverbrauch führen.

Weiterhin ist aus der AT 001 235 U1 ein Lichttherapiegerät für eine Behandlung des menschlichen Körpers über dessen Auge bekannt. Das Gerät besteht dabei aus einem brillen- bzw. augenbindenartig um den Kopf legbaren Gehäuse, das ein Lichtbündel emittiert und mittels einer Tragevorrichtung für den Kopf vor den Augen des Behandelnden befestigt werden kann. Derartige Kopfgeräte haben jedoch wenig Resonanz gefunden, da sie unbequem zu tragen sind und Therapiesitzungen mühsam machen.

Aus der FR 26 39 239 ist eine Lichttherapievorrichtung zur Behandlung von Hautkrankheiten, Narben, Wundliegen Rheumatismus und Neuralgie bekannt die sichtbares Licht ohne ultravioletten und infraroten Strahlungsanteil auf die nackte Haut des Patienten strahlt. Hierzu ist eine sechseckige, geschlossene Lichtkabine vorgesehen, in die das Licht über punktförmige Lichtquellen, die von den Enden von Lichtfaserkabeln gebildet werden und an den Wänden der Kabine verteilt angeordnet sind, eingestrahlt wird. Die Innenwände der Kabine sind hierbei verspiegelt, so dass die eingeleiteten Lichtstrahlen an den Wänden reflektiert werden

Die EP 11 55 713 beschreibt ein paneelförmiges Lichttherapiegerät, bei dem in einem Fokussierbereich, auf den der Anwender seine Augen richten soll, die Helligkeit des Leuchtpaneels reduziert sein soll um eine komfortablere Anwendung zu erreichen. Weiterhin beschreibt die DE 44 33 291 eine Therapieleuchte gegen Winterdepression, deren Leuchtstofflampen rückseitig von rinnenförmigen Spiegelreflektoren umgeben sind und deren Vorderseite durch Licht streuende Scheiben abgedeckt sind. Ferner beschreibt die DE 196 06 396 A1 eine Sekundärspiegelreflektoranordnung zur Lichttherapie und UV-Bestrahlung, bei der das Licht von Leuchtstofflampen durch Hilfsreflektoren gebündelt auf parabolisch ausgebildete Sekundärspiegelreflektoren gelenkt und von diesen auf das Auge des Patienten fokussiert wird.

Hiervon ausgehend liegt der vorliegenden Erfindung die Aufgabe zugrunde, eine verbesserte Lichttherapievorrichtung der genannten Art zu schaffen, die Nachteile des Standes der Technik vermeidet und letzteren in vorteilhafter Weise weiterbildet. Vorzugsweise soll eine effiziente Beeinflussung des Serotoninspiegels ohne Nebenwirkungen mit zeitlich kurzen Therapiesitzungen erreicht werden.

Erfindungsgemäß wird diese Aufgabe durch eine Lichttherapievorrichtung gemäß Anspruch 1 gelöst. Bevorzugte Ausgestaltungen der Erfindung sind Gegenstand der abhängigen Ansprüche.

Die Erfindung geht von dem Gedanken aus, dass durch eine ausreichende Vergrößerung des Raumwinkelbereichs, über den hinweg die Pupille das Therapielicht wahrnehmen bzw. aufnehmen kann, sowie eine Vergleichmäßigung der Lichtintensität bzw. Leuchtdichte über einen ausreichend großen Raumwinkelbereich hinweg im Vergleich zu monitorartigen Geräten mit wesentlich verringerten Leuchtdichten gearbeitet und dennoch auch bei kurzen Therapiesitzungen eine effiziente Beeinflussung des Serotoninspiegels erreicht werden kann. Der Zeitanteil, während dem die Pupille an der Lichtquelle vorbeischaut, wird kleiner, während der Zeitanteil einer Therapiesitzung, in dem das Therapielicht auf die Pupille trifft, größer wird, so dass ein besserer Wirkungsgrad auch bei niedrigeren Leuchtdichten erreicht wird. Hier wird durch die Synchronisation der Leuchtdichte des Geräts mit der Umgebungshelligkeit der optische Wahrnehmungsvorgang nicht gestört, was andernfalls über den Adaptionsvorgang des visuellen Systems an den Hell-/Dunkelwechsel der Fall wäre. Dementsprechend können Kopfschmerzen und andere Nebenwirkungen vermieden werden. Erfindungsgemäß zeichnet sich die Lichttherapievorrichtung dadurch aus, dass die Lichtabgabevorrichtung eine Lichtkabine mit einer als Leuchte ausgebildeten Kabinenwand umfasst, die das abgegebene Licht auf ein Kabinenzentrum richtet, in dem ein Therapieplatz vorgesehen ist. Insbesondere kann im Kabinenzentrum dabei ein Sitzplatz und/oder ein Lese- bzw. Arbeitsplatz vorgesehen sein, um eine angenehme und entspannte Therapiesitzung zu ermöglichen. Die Verwendung einer im Vergleich zu bisherigen Tischgeräten sehr viel größeren Lichtkabine erlaubt bei abgesenkten Leuchtdichten unter Beibehaltung kurzer Therapiezeiten eine effiziente Beeinflussung des Serotoninspiegels ohne spürbare Nebenwirkungen. Dabei tritt praktisch keine Blendwirkung auf, da die Leuchtdichten im erträglichen Bereich gehalten werden können. Zudem hat eine Blickänderung nur wenig Folgewirkung, da das Therapielicht dann eben aus einer anderen Richtung auf die Pupille trifft. Dies ermöglicht auch andere Tätigkeiten während der Therapiesitzung wie Lesen oder Arbeiten. Insgesamt ergibt sich ein hoher Wirkungsgrad bei geringem Energiebedarf.

Erfindungsgemäß umschließt die Lichtkabine vom Kabinenzentrum bzw. dem dort vorgesehenen Therapieplatz aus betrachtet ein Sichtfeld mit einem Raumwinkelbereich von zumindest ¾ π bis 2 π. Insbesondere kann vom Therapieplatz aus betrachtet ein Raumwinkelbereich von mehr als 1 π von der als Leuchte ausgebildeten Kabinenwand umschlossen sein. Eine Lichtfläche über einen Raumwinkelbereich von mehr als 1 n erlaubt eine effiziente Beaufschlagung der Pupille mit Licht auch bei entspanntem Sehen mit hin-und hergehendem Blick. Vorteilhafterweise erfolgt die Beleuchtung des genannten Raumwinkelbereichs dabei von allen Raumrichtungen her, so dass eine Lichteinstrahlung auf die Pupille auch bei nach oben bzw. nach unten gerichtetem Blick erfolgt.

Die Lichtkabine selbst kann dabei grundsätzlich verschiedene Formen besitzen. In Weiterbildung der Erfindung kann die Lichtkabine vorteilhafterweise einen etwa igluförmigen Halbraum begrenzen, der zu einer Seite hin offen ist, so dass der Therapieplatz im Zentrum des genannten Halbraums einfach betreten werden kann. Insbesondere kann in Weiterbildung der Erfindung die Lichtkabine einen stehenden bogenförmigen, vorzugsweise etwa ein Halbrund bildenden, Wandteil aufweisen, der von einer Decke und einem Boden oben und unten begrenzt wird. Die Decke, ggf. auch der Boden, kann dabei grundsätzlich eine schalenförmige Wölbung besitzen, so dass sich insgesamt eine viertel- oder gar halbkugelförmige, kathedralenartige Gestalt für den von der Lichtkabine begrenzten Innen- bzw. Therapieraum ergibt. In Weiterbildung der Erfindung können im Sinne einer einfachen Ausbildung die Decke und der Boden jedoch auch eben ausgebildet sein, so dass der stehende, ein Halbrund bildende Wandteil oben und unten von ebenen Flächen begrenzt wird. Der Boden kann hierbei als Sockel ausgebildet sein, vorteilhafterweise etwa auf Tischniveau oder geringfügig unterhalb, so dass insgesamt eine geringere Fläche im Bereich des halbrunden Wandteils zu beleuchten ist.

Vorteilhafterweise sind die Decke und der Boden verspiegelt ausgebildet. Grundsätzlich könnte auch eine aktiv leuchtende Ausbildung von Decke und Boden vorgesehen sein. Ausgehend von einem Therapieplatz in Form eines Sitzplatzes ist es jedoch ausreichend, wenn nur der stehende, bogenförmige Wandteil der Lichtkabine aktiv Licht abgibt. Die reflektierende Ausbildung von Decke und/oder Boden reicht aus, um eine gleichmäßige Leuchtdichte über den zuvor genannten Raumwinkelbereich zu erzielen.

Der stehende, bogenförmige Wandteil der Lichtkabine kann grundsätzlich eine durchgängig bogenförmig gewölbte Lichtfläche bilden. In Weiterbildung der Erfindung jedoch ist der genannte bogenförmige stehende Wandteil segment- bzw. polygonzugartig aus mehreren Leuchtpaneelen zusammengesetzt, die für sich betrachtet eine im wesentlichen ebene Oberfläche haben können, nebeneinander angeordnet jedoch insgesamt ein polygonzugartiges Halbrund bilden. Dies erleichtert einerseits die Fertigung und Montage der Lichtkabine, andererseits gibt sie der den sichtbaren Raum begrenzenden Lichtfläche eine gewisse Struktur, was ein ermüdungsfreies Sehen durch eine erleichterte Orientierung im Lichtkabinenraum ermöglicht.

Je nach Anwendungszweck kann die in der Lichtkabine, insbesondere im Bereich des Kabinenzentrums bereitgestellte Leuchtdichte variieren, um beispielsweise je nach Patientenempfindlichkeit unterschiedlich lange Therapiesitzungen vorsehen zu können. Dabei ist die Lichtabgabevorrichtung derart ausgebildet, dass für eine Position im Bereich des Kabinenzentrums Leuchtdichten zwischen 150 und 4000 cd/m², vorzugsweise 250 bis 3000 cd/m², und insbesondere etwa 400 bis 2000 cd/m² sichtbar sind. Bereits im Bereich um die 1000 cd/m² herum, d.h. von etwa 800 bis 1200 cd/m², können beeindruckende Ergebnisse hinsichtlich der Beeinflussung des Serotoninspiegels erzielt werden, wobei aufgrund der geringeren Leuchtdichte im Vergleich zu vormaligen Tischgeräten Nebenwirkungen wie Kopfschmerzen, Ermüdungserscheinungen und mentalen, physiologischen Belastungen wie Stress, sicher vermieden werden.

Die Größe der Lichtkabine kann je nach Anwendungsfall variieren, beispielsweise können kleinere, ggf. noch auf einem Tisch plazierbare Ausführungen mit einem Durchmesser von etwa einem Meter vorgesehen werden. Bevorzugt ist jedoch eine auf dem Fußboden stehende Lichtkabine mit einem Durchmesser zwischen zwei und vier Metern, insbesondere etwa drei Metern, was sich als sehr guter Kompromiss zwischen effektiver, nebenwirkungsfreier Therapie einerseits und raumsparender, auch in kleineren Praxen noch handhabbarer Aufstellbarkeit andererseits gezeigt hat.

Um eine stabile Wahrnehmung zu ermöglichen, die keinen Adaptionsvorgang des visuellen Systems erfordert, bilden die Kabinenwände eine vollständig beleuchtete, im wesentlichen gleichmäßig helle Leuchtfläche frei von dunklen Bereichen, die vom Kabinenzentrum aus betrachtet ein Sichtfeld mit einem Raumwinkelbereich von mehr als ¾ n vollständig abdeckt.

Um ein entspanntes Sehen bei gleichzeitig hoher Effizienz der Lichttherapie zu erreichen, sind die Kabinenwände als virtuelle Wand ausgebildet, die kein Begrenzungsempfinden vermittelt. Dabei weisen die als Leuchte ausgebildeten Kabinenwandflächen eine optisch wirksame Abstrahlfläche mit Richt-und Streumitteln auf, die eine gerichtete Lichtabstrahlung mit einer gewissen, jedoch nur teilweisen Streuung des abgestrahlten Lichts bewirken. Die genannten Richt- und Streumittel bewirken also eine Mischung aus gerichteter und diffuser Lichtabstrahlung. Da einerseits keine gänzlich diffuse Lichtabgabe vorgesehen ist, wird ein gewisses Empfinden der Orientierung vermittelt, zudem kann eine höhere Effizienz bei der Lichtabgabe erreicht werden. Andererseits bewirkt eine gewisse Diffusität der Lichtabgabe ein warmes, angenehmes Empfinden der Lichttherapie und eine gleichmäßige Leuchtdichte.

Die Richt- und Streumittel an den Abstrahl- und/oder Austrittsflächen an den Kabinenwänden können in Weiterbildung der Erfindung einen Aufstreuwinkel von 5° bis 40°, vorzugsweise etwa 10° bis 30°, vorsehen. Eine solche begrenzte Aufstreuung kann beispielsweise durch eine Oberflächenstrukturierung auf einer Reflektorfläche und/oder einer Abdeckscheibe, durch die das Licht austritt, erreicht werden. Beispielsweise kann die Oberflächenstrukturierung einen spiegelnden Anteil und einen leicht mattierten Anteil aufweisen, so dass Spiegelbilder leicht verwischt, eine gänzlich diffuse Lichtabgabe jedoch vermieden wird.

In Weiterbildung der Erfindung bestehen die Kabinenwände, zumindest die stehend angeordneten Wandteile, aus Leuchtpaneelen, die jeweils zumindest eine Lampe, einen die Lampe zum Kabinenzentrum hin abschattenden Abschatter, einen zum Kabinenzentrum hin gerichteten Reflektor sowie eine vor dem Reflektor sitzende Abdeckscheibe aufweist. Die Lampe ist dabei vorteilhafterweise vom Kabinenzentrum aus nicht sichtbar. Das von ihr abgegebene Licht wird indirekt über die Reflektorfläche zum Kabinenzentrum hin abgestrahlt. Vorteilhafterweise kann ein Leuchtpaneel auch mehrere Lampen aufweisen, die beispielsweise verschiedene Farben aufweisen können, um eine entsprechende Farbmischung zu erzielen. Vorteilhafterweise sind die mehreren Lampen dabei in Nachbarschaft zueinander angeordnet, wobei sie vorteilhafterweise zum Kabinenzentrum hin von einem gemeinsamen Abschatter abgeschattet sein können.

Die Reflektoren der Leuchtpaneele können hierbei grundsätzlich verschieden ausgebildet sein, beispielsweise kontinuierlich gekrümmte Reflektionsflächen besitzen. Vorteilhafterweise jedoch sind die Reflektoren als Rinnenreflektoren ausgebildet, deren Rinnenreflektionsflächen auf das Kabinenzentrum hin gerichtet sind. Derartige Rinnenreflektoren geben bei einfacherer Herstellung dem abgegebenen Licht eine gewisse Struktur, die bei der Lichttherapie als angenehm empfunden wird.

Die vor dem Reflektor sitzende Abdeckscheibe besitzt vorteilhafterweise eine Lichtdurchlässigkeit τ im Bereich von 0,5 bis 1. Die dem abgegebenen Licht eine begrenzte Aufstreuung gebende Oberflächenstruktur kann auf der genannten Abdeckscheibe vorgesehen sein. Alternativ oder zusätzlich kann jedoch auch die Oberfläche des Reflektors mit einer entsprechenden Strukturierung versehen sein.

Die Erfindung wird nachfolgend anhand eines bevorzugten Ausführungsbeispiels und zugehöriger Zeichnungen näher erläutert. In den Zeichnungen zeigen:

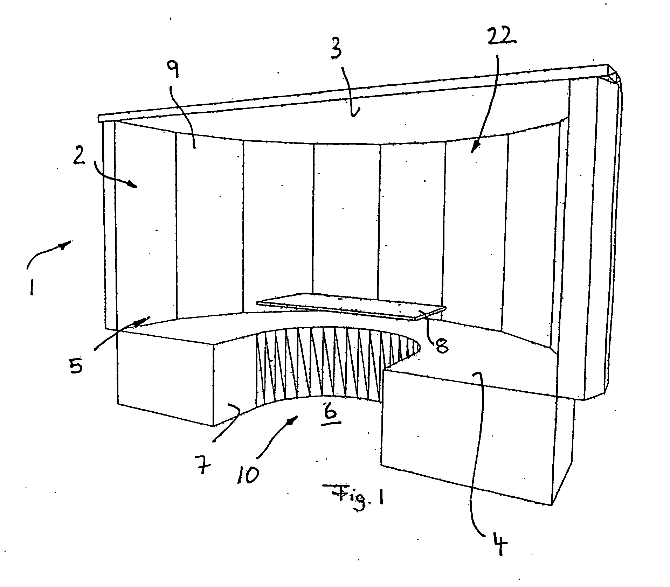

- Fig. 1:: eine perspektivische Ansicht einer Lichttherapiekabine nach einer vor- teilhaften Ausführung der Erfindung schräg von vorne,

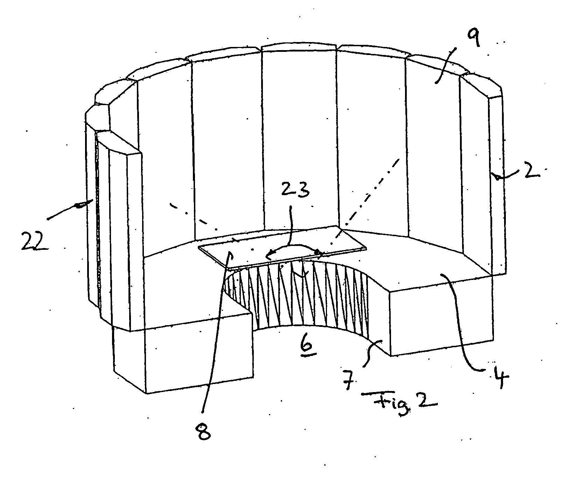

- Fig. 2:: eine perspektivische Ansicht der Lichttherapiekabine aus Fig. 1 ohne Decke aus einem anderen Blickwinkel,
- Fig. 3:: eine Draufsicht auf die Lichttherapiekabine aus Fig. 2 von oben, die die halbkreisförmige Außenkontur der Lichttherapiekabine und deren Wände aus einzelnen Lichtpaneelen zeigt,
- Fig. 4:: einen Querschnitt durch ein Leuchtpaneel, das einen Teil der Wandung der Lichttherapiekabine bildet, und
- Fig. 5:: einen Querschnitt durch ein Leuchtpaneel ähnlich Fig. 4, das anstelle der in Fig. 4 gezeigten einen Lampe drei Lampen aufweist.

Die in den Figuren gezeichnete Lichttherapiekabine 1 begrenzt mit ihren Kabinenwänden 2, 3 und 4 einen - grob gesprochen - theaterartigen, halbrunden und zu einer Seite offenen Innenraum 5, in dem ein Therapieplatz 6 vorgesehen ist. In der gezeichneten Ausführungsform umfassen die genannten Kabinenwände dabei einen stehenden, bogenförmigen bzw. etwa halbkreisförmigen Wandteil 2, an den eine den Innenraum 5 nach oben hin begrenzende Decke 3 sowie ein den Innenraum 5 nach unten hin begrenzender Boden 4 angrenzen. Dabei sind in der gezeichneten Ausführung die Decke 3 sowie der Boden 4 jeweils eben ausgebildet, wobei der Boden 4 in der gezeichneten Ausführung in Form eines erhöht angeordneten Sockels ausgebildet ist und zentral eine buchtförmige Aussparung 7 aufweist, in der der Therapieplatz 6 angeordnet ist. Letztgenannter Therapieplatz 6 ist vorteilhafterweise mit einem nicht eigens gezeigten Stuhl oder anderweitigem Sitzplatz sowie einer Tischfläche 8 versehen, so dass am Therapieplatz 6 gelesen oder auch gearbeitet werden kann.

Der Therapieplatz 6 ist im wesentlichen im Kabinenzentrum angeordnet, auf das hin die Kabinenwände 2, 3 und 4 mit jeweils einer Hauptfläche ausgerichtet sind.

Die Decke 3 sowie der Boden 4 sind vorteilhafterweise verspiegelt ausgebildet bzw. mit einer Verspiegelung versehen. Beispielsweise kann die Decke 3 mit einem Spiegelblech kaschiert sein. Die obere Fläche des Sockels des Bodens 4 kann eine entsprechende Verspiegelung aufweisen.

Der aufrechte, halbbogenförmige Kabinenwandteil 2 besteht vorteilhafterweise aus einer Mehrzahl, vorzugsweise zumindest drei, besser noch mehr als fünf und in der gezeichneten Ausführung aus insgesamt neun Leuchtpaneelen 9, die insgesamt betrachtet jeweils eine rechteckige Kontur besitzen und hochkant angeordnet sind. Die dem Kabinenzentrum 10 zugewandten Oberflächen der Leuchtpaneele 9 sind eben ausgebildet und insbesondere von einer Abdeckscheibe gebildet.

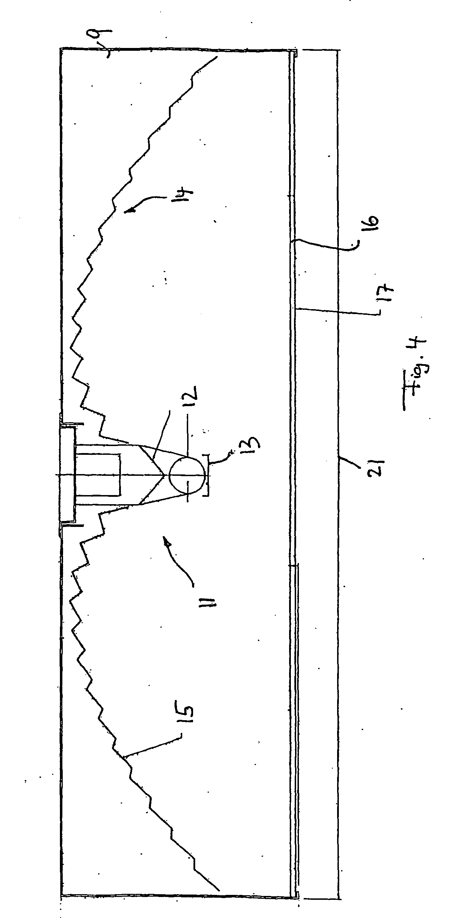

Wie Figur 4 zeigt, umfasst jedes Leuchtpaneel 9 ein optisches System 11 zur aktiven Abgabe von Licht, das unter einer gewissen Aufstreuung auf das Kabinenzentrum 10 hin gerichtet abgegeben wird. Das optische System 11 umfasst in der gezeichneten Ausführung eine Lampe 12, die zum Therapieplatz 6 hin von einem als Blech ausgebildeten Abschatter 13 abgedeckt wird, so dass das von der Lampe 12 abgegebene Licht nicht direkt auf den Therapieplatz 6 gestrahlt wird bzw. die Lampe 12 selbst vom Therapieplatz 6 aus nicht zu sehen ist. Vorteilhafterweise besteht die Lampe 12 aus einer länglichen, hochkant stehenden Leuchtstofflampe. Alternativ können auch mehr als eine solche Lampe 12 im Zentrum des Leuchtpaneels 9 angeordnet sein, wobei dann vorteilhafterweise der Abschatter 13 größer ausgebildet ist, um gemeinsam alle Lampen 12 abzudecken. Als Lampen können dabei Leuchtstoffröhren, Glühlampen, LEDs und LED-Anordnungen sowie andere geeignete Lichtquellen Verwendung finden.

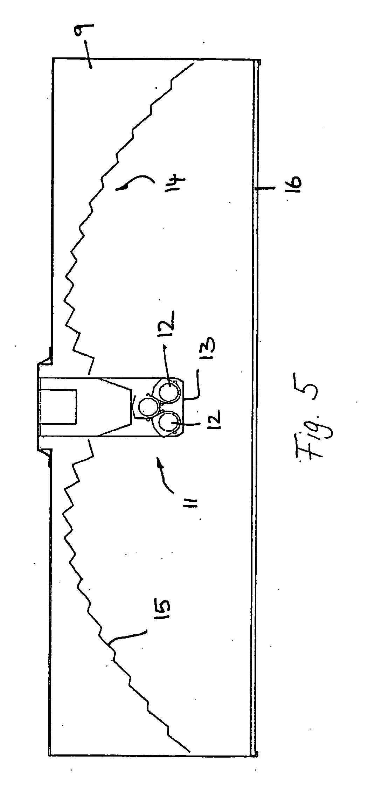

Wie Figur 5 zeigt, kann das optische System 11 der Leuchtpaneele 9 auch mehrere Lampen 12 umfassen, mittels derer vorteilhafterweise auch eine Farbmischung erreicht werden kann. Wie Figur 5 zeigt, sind die mehreren Lampen 12 vorteilhafterweise in Nachbarschaft zueinander angeordnet, wobei ein gemeinsamer Abschatter 13 vorgesehen sein kann, der die mehreren Lampen 12 zum Therapieplatz 6 hin abdeckt.

Im Bereich der Rückseite des Leuchtpaneels 9 umfasst letzteres einen als Rinnenreflektor ausgebildeten Reflektor 14, dessen Reflektionsflächen 15 hochkant stehende, streifenförmige Flächenabschnitte bilden, die verschieden geneigt angeordnet sind derart, dass das von der Lampe 12 kommende Licht zum Therapieplatz 6 hin abgegeben wird, vgl. Figur 4 und Figur 5.

Auf der Vorderseite des Leuchtpaneels 9 ist eine Abdeckscheibe 16 vorgesehen, durch die hindurch das vom Reflektor 14 reflektierte Licht austritt.

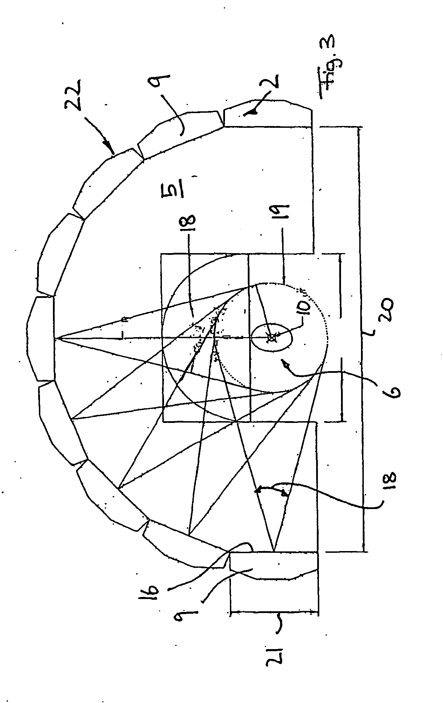

Vorteilhafterweise sind dem Reflektor 14 und/oder der Abdeckscheibe 16 Streumittel 17 in Form einer Oberflächenstrukturierung zugeordnet, durch die eine gewisse, jedoch begrenzte Aufstreuung des abgegebenen Lichts bewirkt wird. Wie Figur 3 zeigt, besitzt das optische System 11 eines jeden Leuchtpaneels 9 einen Streuwinkel 18 von etwa 30°. Der Streuwinkel und die Größenabmessungen der Lichtkabine 1 bzw. die Positionierung des Therapieplatzes 6 sind vorteilhafterweise derart aufeinander abgestimmt, dass im Kabinenzentrum 10 ein mit gleichmäßiger Leuchtdichte beleuchteter Bewegungsbereich 19 bestrahlt wird, innerhalb dessen eine zu therapierende Person ohne Beeinträchtigung der Lichtbeaufschlagung sich hin- und herbewegen kann. In der gezeichneten Ausführung umfasst der genannte Bewegungsbereich 19, der sich als Hüllkurve in die Vielzahl der Streuwinkel einschmiegt, einen Durchmesser von etwa 0,5 bis 1,0 m.

Die Lichtkabine 1 kann einen Durchmesser 20 von etwa 2 bis 4 m umfassen, wobei der Durchmesser 20 in der gezeichneten Ausführung etwa 3 m beträgt. Der Abstand des Therapieplatzes 6 von den Kabinenwänden 2 beträgt vorteilhafterweise etwa zwischen 1 und 2 m, insbesondere etwa 1,5 m. Bezüglich der Dimension der Leuchtpaneele 9 sind verschiedene Variationen möglich, wobei sich deren Höhe vorteilhafterweise zwischen 1 und 2 m, insbesondere etwa im Bereich um 1,5 m bewegt. Die Breite 21 der Leuchtpaneele 9 beträgt vorteilhafterweise zwischen 30 und 100 cm, insbesondere etwa 60 cm. Es versteht sich jedoch, dass weitere Variationen in Anpassung an die übrigen Geometrieverhältnisse möglich sind.

## Patentansprüche

1. Lichttherapievorrichtung zur Beeinflussung des Serotoninspiegels, mit einer Lichtabgabevorrichtung (22) zur Abgabe von über das menschliche/tierische Auge aufnehmbaren Lichts, wobei die Lichtabgabevorrichtung (22) eine Lichtkabine (1) mit einer als Leuchte ausgebildeten Kabinenwand (2, 3, 4) umfasst, die das abgegebene Licht auf ein Kabinenzentrum (10) richtet, in dem ein Therapieplatz (6) vorgesehen ist, wobei die Kabinenwand (2, 3, 4) vom Therapieplatz (6) aus betrachtet ein Sichtfeld über einen Raumwinkel von zumindest 3/4 π umschließt, **dadurch gekennzeichnet, dass** die Kabinenwand (2, 3, 4) eine gleichmäßig leuchtende Leuchtfläche frei von dunklen Bereichen bildet, die das vom Therapieplatz (6) aus bestimmungsgemäß betrachtete Sichtfeld im Abstand von 1 m bis 3m vom Therapieplatz (6) über den genannten Raumwinkel (23) vollständig abdeckt und eine optisch wirksame Abstrahlfläche (14, 16) mit Richt- und Streumitteln (17) aufweist, die eine gerichtete Lichtabstrahlung mit einer gewissen, nur teilweisen Streuung des abgegebenen Lichts vorsehen derart, daß im Bereich des Therapieplatzes (3) Leuchtdichten zwischen 150 und 4000 cd/m² sichtbar sind.

2. Lichttherapievorrichtung nach dem vorhergehenden Anspruch, wobei im Kabinenzentrum (10) ein Sitzplatz und/oder eine Lese- und/oder Arbeitstischfläche (8) vorgesehen ist.

3. Lichttherapievorrichtung nach einem der vorhergehenden Ansprüche, wobei die als Leuchte ausgebildete Kabinenwand (2, 3, 4) vom Kabinenzentrum (10) aus betrachtet ein Sichtfeld mit einem Raumwinkel (23) π bis 2 π umschließt.

4. Lichttherapievorrichtung nach einem der vorhergehenden Ansprüche, wobei der Therapieplatz (6) von der Kabinenwand (2, 3, 4) einen Abstand von 1 bis 2 m, und insbesondere etwa 1,5 m, aufweist.

5. Lichttherapievorrichtung nach einem der vorhergehenden Ansprüche, wobei die Lichtkabine (1) einen Durchmesser (20) von zumindest 1 m, vorzugsweise zwischen 2 bis 4 m, aufweist.

6. Lichttherapievorrichtung nach einem der vorhergehenden Ansprüche, wobei die Lichtkabine (1) einen stehenden bogenförmigen, vorzugsweise etwa ein Halbrund bildenden, Wandteil (2) aufweist, der zusammen mit einer Decke (3) und einem Boden (4) einen Kabineninnenraum (5) begrenzt.

7. Lichttherapievorrichtung nach dem vorhergehenden Anspruch, wobei der stehende Wandteil (2) segment- oder polygonzugartig aus mehreren Leuchtpaneelen (9) zusammengesetzt ist und/oder die Decke und/oder der Boden (4) verspiegelt ausgebildet sind.

8. Lichttherapievorrichtung nach einem der vorhergehenden Ansprüche, wobei die Lichtabgabevorrichtung (22) derart ausgebildet ist, dass für eine Position im Bereich des Therapieplatzes (3) im Kabinenzentrum (10) Leuchtdichten zwischen 250 bis 3000 cd/m², insbesondere etwa 400 bis 2000 cd/m², sichtbar sind.

9. Lichttherapievorrichtung nach einem der vorhergehenden Ansprüche, wobei die Richt- und Streumittel einen Aufstreuwinkel (18) von 5° bis 40°, vorzugsweise 10° bis 30°, vorsehen.

10. Lichttherapievorrichtung nach einem der vorhergehenden Ansprüche, wobei die Richt- und Streumittel (17) eine Oberflächenstrukturierung auf einer Reflektorfläche und/oder einer Abdeckscheibe (16) aufweisen.

11. Lichttherapievorrichtung nach einem der vorhergehenden Ansprüche, wobei als Kabinenwände Leuchtpaneele (9) vorgesehen sind, die eine Lampe (12), einen die Lampe (12) zum Therapieplatz (6) hin abschattenden Abschatter (13), einen zum Kabinenzentrum (10) hin gerichteten Reflektor (14) sowie eine vor dem Reflektor (14) angeordnete Abdeckscheibe (16) aufweist.

12. Lichttherapievorrichtung nach dem vorhergehenden Anspruch, wobei mehrere Lampen (12) an einem Leuchtpaneel vorgesehen sind.

13. Lichttherapievorrichtung nach einem der beiden vorhergehenden Ansprüche, wobei der Reflektor (14) als Rinnenreflektor ausgebildet ist, dessen Rinnenreflektionsflächen (15) auf das Kabinenzentrum (10) gerichtet sind.

14. Lichttherapievorrichtung nach einem der drei vorhergehenden Ansprüche, wobei die Abdeckscheibe (16) eine Oberflächenstruktur in Form von vorzugsweise aufrecht verlaufenden Rillen aufweist.

## Claims

1. Light therapy device for impacting serotonin levels with a light emission device (22) for the emission of light that can be absorbed by the eyes of a human / animal, wherein the light emission device (22) comprises a light cabin (1) with a cabin wall (2, 3, 4) constructed as a light which directs the emitted light on to a cabin centre (10) into which a therapy area is provided (6), wherein the cabin wall (2, 3, 4), from the therapy area (6) outwards, has a field of vision around itself with a solid angle of at least 3/4π, **characterised in that**, the cabin wall (2, 3, 4) forms an evenly lit lighting area free from dark areas, completely covering the intended field of vision from the therapy area (6) at a distance of 1 m to 3m from the therapy area (6) to the aforementioned solid angle (23) and features an optically effective radiating surface (14, 16) with directional and spreading means (17) providing a directed beam of light with a certain but only partial scattering of the emitted light in such a way that luminances between 150 and 4000 cd/m² are visible in the therapy area (3).

2. Light therapy device according to the preceding claim, wherein a seat and/or a reading and or work surface (8) is provided in the cabin centre (10).

3. Light therapy device according to any one of the preceding claims, wherein the cabin wall (2, 3; 4) from the cabin centre (10) outwards, constructed as a light, has a field of vision around itself with a solid angle (23) π to 2 π.

4. Light therapy device according to any one of the preceding claims, wherein the therapy area (6) is at a distance of 1 to 2 m and in particular around 1.5 m from the cabin wall (2, 3, 4).

5. Light therapy device according to any one of the preceding claims, wherein the light cabin (1) has a diameter (20) of at least 1 m, preferably 2 to 4 m.

6. Light therapy device according to any one of the preceding claims, wherein the light cabin (1) has a vertical arched or preferably something like a semicircular wall panel (2) which limits the cabin interior (5) along with a ceiling (3) and a floor (4).

7. Light therapy device according to the preceding claim, wherein the segmental or polygonal vertical wall panel (2) is composed of several light panels (9) and/or the ceiling and/or the floor (4) are designed to be mirrored.

8. Light therapy device according to one of the preceding claims wherein the light emission device (22) is designed in such a way that for a position in the therapy area (3) in the cabin centre (10) luminances between 250 to 3000 cd/m², in particular around 400 to 2000 cd/m², are visible.

9. Light therapy device according to one of the preceding claims wherein the directional and spreading means provide for a scattering angle (18) of 5° to 40°, preferably 10° to 30°.

10. Light therapy device according to one of the preceding claims, wherein the directional and spreading means (17) feature an upper surface structure on a reflector surface and/or a covering screen (16).

11. Light therapy device according to one of the preceding claims, wherein light panels (9) are provided as cabin walls featuring a lamp (12), a lamp (12) in the therapy area (6), a shielding screen (13), a reflector (14) that is directed into the cabin centre (10) and a covering screen (16) that is placed in front of the reflector (14).

12. Light therapy device according to the preceding claim, wherein several lamps (12) are provided on a lighting panel.

13. Light therapy device according to one of the two preceding claims, wherein the reflector (14) is designed as a trough-shaped reflector of which the reflector surfaces (15) are directed on to the cabin centre (10).

14. Light therapy device according to one of the three preceding claims, wherein the covering screen (16) features an upper surface structure preferably in the shape of perpendicular running grooves.

## Revendications

1. Dispositif de luminothérapie destiné à influencer le niveau de sérotonine, avec un dispositif de distribution de la lumière (22) destiné à la distribution de lumière absorbable via l'oeil humain/animal, le dispositif de distribution de la lumière (22) comprenant une cabine lumineuse (1) avec une paroi de cabine formée en dispositif d'éclairage (2, 3, 4), qui dirige la lumière délivrée vers un centre de cabine (10), dans lequel une place de thérapie (6) est prévue, la paroi de cabine (2, 3, 4) vue de la place de thérapie (6) entourant un champ de vision via un angle solide d'au moins 3/4 π, **caractérisé en ce que** la paroi de cabine (2, 3, 4) constitue une surface d'éclairage rayonnant de manière régulière sans zones sombres, qui recouvre complètement le champ de vision vu conformément à sa disposition de la place de thérapie (6) dans un espace de 1 m à 3 m de la place de thérapie (6) via ledit angle solide (23) et présente une surface de rayonnement active optiquement (14, 16) avec des moyens de mise au point et de diffusion (17), qui prévoient un rayonnement lumineux mis au point avec une certaine diffusion, seulement partielle de la lumière distribuée de sorte que, dans la zone de la place de thérapie (3), des luminances de 150 à 4000 cd/m² sont visibles.

2. Dispositif de luminothérapie selon la revendication précédente, une place assise et/ou une surface de lecture et/ou de bureau (8) étant prévue au centre de cabine (10).

3. Dispositif de luminothérapie selon une quelconque des revendications précédentes, la paroi de cabine formée en dispositif d'éclairage (2, 3, 4) entourant, vue du centre de cabine (6), un champ de vision via un angle solide (23) de π à 2 π.

4. Dispositif de luminothérapie selon une quelconque des revendications précédentes, la place de thérapie (6) présentant de la paroi de cabine (2, 3, 4) un espace de 1 à 2 m, et notamment d'environ 1,5 m.

5. Dispositif de luminothérapie selon une quelconque des revendications précédentes, la cabine lumineuse (1) présentant un diamètre (20) d'au moins 1 m, de préférence de 2 à 4 m.

6. Dispositif de luminothérapie selon une quelconque des revendications précédentes, la cabine lumineuse (1) présentant un élément de paroi (2) dressé en voûte, formant approximativement de préférence un demi-cercle, qui, ensemble avec un plafond (3) et un plancher (4) délimite un habitacle de cabine (5).

7. Dispositif de luminothérapie selon la revendication précédente, l'élément de paroi dressé (2) étant composé de plusieurs panneaux lumineux (9) de manière segmentée ou polygonale et/ou le plafond et/ou le plancher (4) étant formés de manière réfléchissante.

8. Dispositif de luminothérapie selon une quelconque des revendications précédentes, le dispositif de distribution de la lumière (22) étant formé de sorte que, pour une position dans la zone de la place de thérapie (3) au centre de cabine (10) des luminances de 250 à 3000 cd/m², notamment d'environ 400 à 2000 cd/m², sont visibles.

9. Dispositif de luminothérapie selon une quelconque des revendications précédentes, les moyens de mise au point et de diffusion prévoyant un angle de diffusion (18) de 5 ° à 40 °, de préférence de 10 ° à 30 °.

10. Dispositif de luminothérapie selon une quelconque des revendications précédentes, les moyens de mise au point et de diffusion (17) présentant une structure superficielle sur une surface de réflecteur et/ou un disque de recouvrement (16).

11. Dispositif de luminothérapie selon une quelconque des revendications précédentes, des panneaux lumineux (9) étant prévus comme parois de cabine, qui présentent une lampe (12), un obscursisseur (13) obscurcissant la lampe (12) vers la place de thérapie (6), un réflecteur (14) dirigé vers le centre de cabine (10) ainsi qu'un disque de recouvrement (16) disposé devant le réflecteur (14).

12. Dispositif de luminothérapie selon la revendication précédente, plusieurs lampes (12) étant prévues sur un panneau lumineux.

13. Dispositif de luminothérapie selon une quelconque des deux revendications précédentes, le réflecteur (14) étant formé en réflecteur de sillon, dont les surfaces de réflexion des sillons (15) sont dirigées vers le centre de cabine (10).

14. Dispositif de luminothérapie selon une quelconque des trois revendications précédentes, le disque de recouvrement (16) présentant une structure superficielle sous forme de sillons se déroulant verticalement de préférence.
